(19) Europäisches Patentamt
European Patent Office
Office européen des brevets

(11) Veröffentlichungsnummer: **0 117 233 B1**

(12) # EUROPÄISCHE PATENTSCHRIFT

(45) Veröffentlichungstag der Patentschrift: **26.08.87**

(51) Int. Cl.⁴: **G 03 C 1/68,** C 09 D 11/10, C 08 F 2/50, C 07 D 295/10, C 07 C 97/10

(21) Anmeldenummer: **84810080.6**

(22) Anmeldetag: **13.02.84**

(54) **Photohärtbare gefärbte Massen.**

(30) Priorität: **18.02.83 CH 903/83**

(43) Veröffentlichungstag der Anmeldung: **29.08.84 Patentblatt 84/35**

(45) Bekanntmachung des Hinweises auf die Patenterteilung: **26.08.87 Patentblatt 87/35**

(84) Benannte Vertragsstaaten: **BE DE FR GB IT NL**

(56) Entgegenhaltungen:
**EP - A - 0 003 002**
**EP - A - 0 007 468**
**DE - A - 3 008 411**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Hüsler, Rinaldo, Dr., Belchenstrasse 12, CH-4054 Basel (CH)**
Erfinder: **Kirchmayr, Rudolf, Dr., Ettingerstrasse 9, CH-4147 Aesch (CH)**
Erfinder: **Rutsch, Werner, Dr., Avenue Weck-Reynold 1, CH-1700 Fribourg (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Aminoalkyl-arylketone und ihre Verwendung als Photoinitiatoren in photohärtbaren gefärbten Massen, die ein olefinisch ungesättigtes Bindemittel und ein Pigment enthalten. Solche Massen werden vor allem als Druckfarben verwendet.

Es ist bekannt, dass man zur Beschleunigung der Photohärtung von gefärbten Massen, wie beispielsweise Druckfarben oder Anstrichstoffen, vor der Bestrahlung Photoinitiatoren zusetzt. Auf diese Weise ist es möglich, solche Massen in sehr kurzer Bestrahlungszeit so weit zu härten, dass ihre Oberfläche nicht mehr klebrig ist. Während es für transparente Überzugsmassen eine Reihe von technisch befriedigenden Photoinitiatoren gibt, stellt die Strahlenhärtung gefärbter Massen wegen der Anwesenheit der Licht-absorbierenden Pigmente ein besonders schwierig zu lösendes Problem dar. Bei Druckfarben kommt dazu die Forderung nach extrem kurzen Härtungszeiten wegen der hohen Geschwindigkeit moderner Druckmaschinen. Die Anforderungen an Photoinitiatoren für gefärbte Massen sind deshalb wesentlich höher als für transparente photohärtbare Massen.

Bisher in der Technik verwendete Photoinitiatoren für die Härtung solcher gefärbter Massen wie z.B. Druckfarben oder Anstrichstoffe sind meist synergistische Gemische von ketonischen Photoinitiatoren mit spezifischen Aminen, beispielsweise Gemische von Benzophenon mit Michler's Keton (4,4'-Bis-dimethylamino-benzophenon) oder mit p-Dimethylaminobenzoesäurealkylestern oder Gemische von Thioxanthonen mit N-Methyl-diethanolamin. Solche Keton-Amin-Gemische neigen zur Vergilbung im Licht. Dies kann sich bereits bei der Strahlenhärtung äussern, spätestens aber bei längerer Lichteinwirkung auf die gehärteten Schichten. Einige dieser Verbindungen sind in den üblichen Acrylharz-Bindemitteln schwer löslich, neigen zur Rekristallisation und verkürzen die Lagerfähigkeit der Gemische beträchtlich. Andere solcher Verbindungen, wie z.B. die Alkanolamine, sind wasserlöslich und daher für Nass-Offset-Druckfarben nicht verwendbar. Ferner wirken die Keton-Amin-Gemische nach einem bimolekularen Startmechanismus, der diffusionskontrolliert ist und daher in höherviskosen Systemen relativ langsam abläuft.

In der EP-A-3002 wurden bereits Verbindungen der Formel

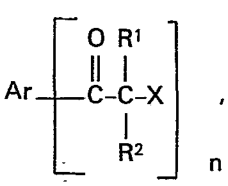,

worin n 1 oder 2 ist, Ar unter anderem ein durch Alkoxy oder Phenoxy substituierter Arylrest sein kann, $R^1$ und $R^2$ $C_1$–$C_8$-Alkyl oder zusammen $C_2$–$C_8$-Alkylen sein können und X eine disubstituierte Aminogruppe sein kann, als Photoinitiatoren vorgeschlagen. Diese Aminoketone stellen also eine molekulare Kombination von Arylketonen und Aminen dar. Die dort beschriebenen Aminoketone erwiesen sich jedoch in Klarlacken den dort ebenfalls beschriebenen Hydroxyketonen (X = OH) wirkungsmässig unterlegen.

Überraschenderweise wurde nun gefunden, dass in pigmentierten photohärtbaren Massen – wie sie z.B. für Druckfarben Verwendung finden – eine bestimmte ausgewählte Gruppe der Aminoketone des EP-A-3002 sich als besonders wirksam erweist und in diesen Substraten auch die Wirkung der Hydroxyketone bei weitem übertrifft. Sie zeigen ausserdem die oben geschilderten Nachteile der Keton-Amin-Gemische nicht oder in wesentlich geringerem Ausmass.

Gegenstand der Erfindung sind daher Verbindungen der Formel I,

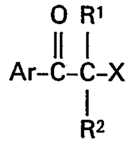 (I)

worin Ar ein in 4-Stellung durch eine Gruppe $-OR^9$ substituierter Phenylrest ist, $R^9$ $C_1$–$C_8$-Alkyl, $C_3$–$C_6$-Alkenyl, Cyclohexyl, Benzyl, Phenyl, Tolyl oder eine der Gruppen $-CH_2CH_2OH$, $-CH_2CH_2-OOC-CH=CH_2$, $-CH_2-COO(C_1-C_4\text{-Alkyl})$, $-CH_2CH_2-COO(C_1-C_4\text{-Alkyl})$,

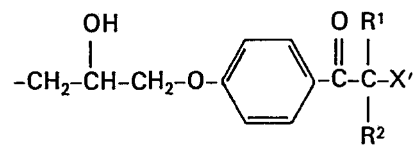

oder 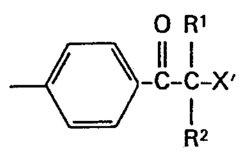

bedeutet, $R^1$ und $R^2$ $C_1$–$C_4$-Alkyl oder $R^1$ und $R^2$ zusammen $C_4$–$C_5$-Alkylen bedeuten und X und X' ein Morpholinorest oder ein Rest der Formel $-N(CH_2CH_2-OCH_3)_2$ sind.

$R^1$ und $R^2$ als Alkyl können z.B. Methyl, Ethyl, Propyl oder Butyl sein. Wenn $R^1$ und $R^2$ zusammen $C_4$–$C_5$-Alkylen bedeuten, so bilden sie zusammen mit dem C-Atom, an das sie gebunden sind, einen Cyclopentan- oder Cyclohexanring.

$R^9$ als Alkyl kann darin unverzweigtes oder verzweigtes Alkyl sein wie z.B. Methyl, Ethyl, Isopropyl, n-Butyl, Isobutyl, tert.-Butyl, Isoamyl, n-Hexyl, n-Octyl oder 2-Ethylhexyl.

$R^9$ als Alkenyl kann insbesondere Alkenyl-methyl sein, wie z.B. Allyl oder Methallyl.

Beispiele für einzelne Verbindungen der Formel I sind folgende Verbindungen:

1-(4-Methoxyphenyl)-2-methyl-2-morpholino-propanon-1

2-Methyl-1-(4-phenoxyphenyl)-2-morpholino-propanon-1
1-(4-Ethoxyphenyl)-2-methyl-2-morpholino-propanon-1
1-(4-Isopropoxyphenyl)-2-methyl-2-morpholino-propanon-1
1-(4-Butoxyphenyl)-2-methyl-2-morpholino-propanon-1
1-(4-Octyloxyphenyl)-2-methyl-2-morpholino-propanon-1
1-[4-(Allyloxy)phenyl]-2-methyl-2-morpholino-propanon-1
1-[4-(2-Butenyloxy)phenyl]-2-methyl-2-morpholino-propanon-1
1-(4-Cyclohexyloxyphenyl)-2-methyl-2-morpholino-propanon-1
1-[4-(2-Hydroxyethoxy)phenyl]-2-methyl-2-morpholino-propanon-1
1-[4-(Benzyloxy)phenyl]-2-methyl-2-morpholino-propanon-1
2-Methyl-1-(4-tolyloxyphenyl)-2-morpholino-propanon-1
1-(4-Anisoyl)-1-morpholino-cyclohexan
1-(4-Ethoxybenzyol)-1-morpholino-cyclohexan
1,3-Bis[4-(α-morpholino-isobutyroyl)phenoxy]-2-hydroxy-propan
4,4′-Bis(α-morpholino-isobutyroyl)-diphenyloxid
1-(4-Methoxybenzoyl)-1-di(2-methoxyyethyl)-amino-cyclohexan
1-(4-Methoxybenzoyl)-1-morpholino-cyclopentan
1-(4-Methoxyphenyl)-2-methyl-2-di(2-methoxyethyl)amino-propanon-1
1-(4-Ethoxyphenyl)-2-methyl-2-di(2-methoxyethyl)amino-propanon-1
1-(4-Methoxyphenyl)-2-methyl-2-morpholino-butanon-1
1-(4-Methoxyphenyl)-2-methyl-2-morpholino-pentanon-1
1-(4-Methoxyphenyl)-2-ethyl-2-morpholino-butanon-1
1-(4-Methoxyphenyl)-2-ethyl-2-morpholino-hexanon-1
1-(4-Methoxyphenyl)-2-propyl-2-morpholino-pentanon-1
1-(4-Methoxyphenyl)-2-methyl-2-di(2-methoxyethyl)amino-butanon-1
1-(4-Methoxyphenyl)-2-methyl-2-di(2-methoxyethyl)amino-pentanon-1
1-(4-Methoxyphenyl)-2-ethyl-2-di(2-methoxyethyl)amino-butanon-1
1-[4-(2-Hydroxyethoxy)phenyl]-2-methyl-2-morpholino-butanon-1
1-[4-(2-Hydroxyethoxy)phenyl]-2-methyl-2-morpholino-pentanon-1
1-[4-(2-Hydroxyethoxy)phenyl]-2-ethyl-2-morpholino-hexanon-1
1-[4-(2-Hydroxyethoxy)benzoyl]-1-morpholino-cyclohexan
1-[4-(2-Hydroxy)phenyl]-2-methyl-2-di(2-methoxyethyl)amino-propanon-1

Bevorzugte Verbindungen sind die Verbindungen der Formeln

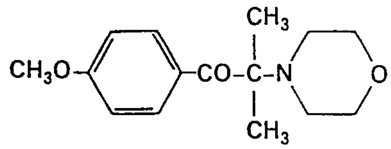

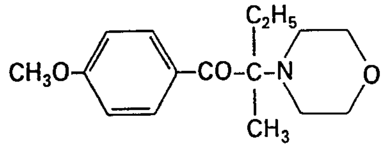

und

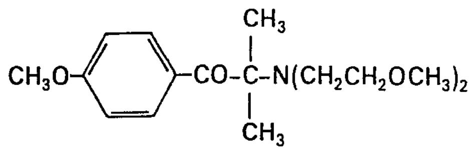

Die Herstellung der Verbindungen der Formel I kann in Analogie zu den aus der EP-A-3002 bekannten Methoden durch Einführung der Aminogruppe in ein entsprechendes Aryl-halogenalkylketon II gemäss folgenden Reaktionsschritten geschehen:

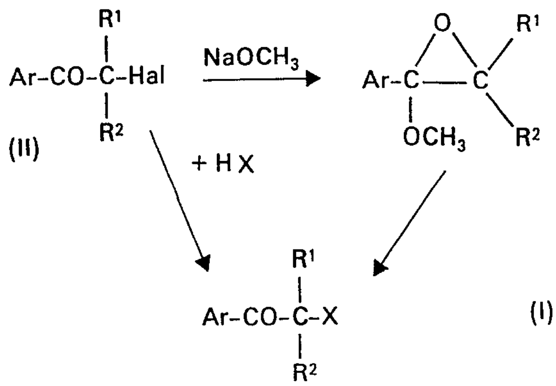


(II) (I)

Hal bedeutet darin Halogen, insbesondere Chlor oder Brom.

Die Halogenalkylketone II können aus den entsprechenden Aryl-alkylketonen Ar-CO-CH($R^1$)($R^2$) nach den üblichen Methoden der α-Halogenierung von Ketonen hergestellt werden. Alternativ kann man sie auch aus den aromatischen Verbindungen ArH durch Friedel-Crafts-Reaktion mit einem α-Halogencarbonsäurehalogenid Hal-C($R^1$)($R^2$)-COHal herstellen.

Wenn Gefahr besteht, dass die Gruppe $R^9$ mit Halogen reagiert, so kann man die Phenolgruppe erst durch eine Schutzgruppe schützen. Nach der Halogenierung und Aminierung wird die Schutzgruppe entfernt und als letzter Schritt wird der Rest $R^9$ eingeführt nach den üblichen Methoden der Veretherung von phenolischen Hydroxylgruppen, vorzugsweise durch Umsetzung mit dem entsprechenden Halogenid $R^9$ Hal.

Wenn $R^9$ $-CH_2CH_2OH$ ist, so wird dieser Rest durch Reaktion mit Ethylenoxid eingeführt. Die Einführung des Restes $-CH_2CH_2COO(C_1-C_4$-Alkyl) kann durch säure- oder basenkatalysierte Addition von Acrylsäureestern geschehen.

Die Verbindungen der Formel I sind ausgezeichnete Photoinitiatoren für die Strahlenhärtung photohärtbarer gefärbter Massen. Die Erfindung betrifft daher auch photohärtbare gefärbte Massen, enthaltend

a) ein olefinisch ungesättigtes photopolymerisierbares Bindemittel,

b) ein Pigment und

c) als Photoinitiator eine Verbindung der Formel I nach Anspruch 1.

Das Bindemittel kann aus einer oder mehreren ungesättigten Verbindungen bestehen, vorzugsweise enthält es zwei oder drei ungesättigte Verbindungen. Daneben kann das Bindemittel noch andere filmbildende Komponenten enthalten, die nicht ungesättigt sind und daher an der Polymerisation nicht teilnehmen. Die ungesättigten Verbindungen können eine oder mehrere olefinische Doppelbindungen enthalten. Sie können niedermolekular (monomer) oder höhermolekular (oligomer) sein. Beispiele für Monomere mit einer Doppelbindung sind Alkyl- oder Hydroxyalkylacrylate oder -methacrylate, wie z.B. Methyl-, Ethyl-, Butyl-, 2-Ethylhexyl- oder 2-Hydroxyethylacrylat, Isobornylacrylat, Methyl- oder Ethylmethycrylat. Weitere Beispiele hierfür sind Acrylnitril, Acrylamid, Methacrylamid, N-substituierte (Meth)acrylamide, Vinylester wie Vinylacetat, Vinylether wie Isobutylvinylether, Styrol, Alkyl- und Halogenstyrole, N-Vinylpyrrolidon, Vinylchlorid oder Vinylidenchlorid.

Beispiele für Monomere mit mehreren Doppelbindungen sind Ethylenglykol-, Propylenglykol-, Neopentylglykol-, Hexamethylenglykol- oder Bisphenol-A-diacrylat, 4,4′-Bis(2-acryloyloxyethoxy)-diphenylpropan, Trimethylol-propan-triacrylat, Pentaerythrit-triacrylat oder -tetraacrylat, Vinylacrylat, Divinylbenzol, Divinylsuccinat, Dilallylphthalat, Triallylphosphat, Triallylisocyanurat oder Tris(2-acryloyloxyethyl)isocyanurat.

Beispiele für höhermolekulare (oligomere) mehrfach ungesättigte Verbindungen sind acrylierte Epoxidharze, acrylierte Polyether, acrylierte Polyurethane oder acrylierte Polyester. Weitere Beispiele für ungesättigte Oligomere sind ungesättigte Polyesterharze, die meist aus Maleinsäure, Phthalsäure und einem oder mehreren Diolen hergestellt werden und Molekulargewichte von etwa 500 bis 3000 besitzen. Solche ungesättigte Oligomere kann man auch als Prepolymere bezeichnen.

Die Bindemittel für die erfindungsgemässen photohärtbaren Massen können z.B. ein Gemisch eines einfach ungesättigten und eines mehrfach ungesättigten Monomeren sein.

Meistens verwendet man jedoch Zweikomponenten-Gemische eines Prepolymeren mit einem mehrfach ungesättigten Monomeren oder Dreikomponentengemische, die ausserdem noch ein einfach ungesättigtes Monomer enthalten. Das Prepolymere ist hierbei in erster Linie für die Eigenschaften des Lackfilmes massgebend, durch seine Variation kann der Fachmann die Eigenschaften des gehärteten Filmes beeinflussen. Das mehrfach ungesättigte Monomere fungiert als Vernetzer, das den Lackfilm unlöslich macht. Das einfach ungesättigte Monomere fungiert als reaktiver Verdünner, mit dessen Hilfe die Viskosität herabgesetzt wird, ohne dass man ein Lösungsmittel verwenden muss.

Solche Zwei- und Dreikomponentensysteme auf der Basis eines Prepolymeren werden sowohl für Druckfarben als auch für Lacke, Photoresists oder andere gefärbte photohärtbare Massen verwendet. Als Bindemittel für Druckfarben werden vielfach auch Einkomponenten-Systeme auf der Basis photohärtbarer Prepolymerer verwendet.

Ungesättigte Polyesterharze werden meist in Zweikomponentensystemen zusammen mit einem einfach ungesättigten Monomer, vorzugsweise mit Styrol, verwendet. Für Photoresists werden oft spezifische Einkomponentensysteme verwendet, wie z.B. Polymaleinimide oder Polychalkone.

Das Bindemittel kann ausserdem nicht-photopolymerisierbare filmbildende Komponenten enthalten. Diese können z.B. physikalisch trocknende Polymere bzw. deren Lösungen in organischen Lösungsmitteln sein, wie z.B. Nitrocellulose oder Celluloseacetobutyrat. Diese können aber auch chemisch bzw. thermisch härtbare Harze sein, wie z.B. Polyisocyanate, Polyepoxide oder Melaminharze. Die Mitverwendung von thermisch härtbaren Harzen ist für die Verwendung in sogenannten Hybrid-Systemen von Bedeutung, die in einer ersten Stufe photopolymerisiert werden und in einer zweiten Stufe durch thermische Nachbehandlung vernetzt werden.

Das in den photohärtbaren Massen enthaltene Pigment kann ein anorganisches Pigment sein, wie z.B. Titandioxid (Rutil oder Anatas), Eisenoxidgelb, Eisenoxidrot, Chromgelb, Chromgrün, Nickeltitangelb, Ultramarinblau, Kobaltblau, Cadmiumgelb, Cadmiumrot oder Zinkweiss. Das Pigment kann ein organisches Pigment sein, wie z.B. ein Mono- oder Bisazopigment oder ein Metallkomplex davon, ein Phthalocyaninpigment, ein polycyclisches Pigment, wie z.B. ein Perylen-, Thioindigo-, Flavanthron-, Chinacridon-, Tetrachlorisoindolinon- oder Triphenylmethan-Pigment. Das Pigment kann auch ein Russ sein oder ein Metallpulver, wie z.B. Aluminium- oder Kupferpulver. Das Pigment kann auch ein Gemisch von zwei oder mehreren verschiedenen Pigmenten sein, wie es zur Erzielung bestimmter Farbtöne üblich ist.

Das Pigment kann in einer Menge von 5 bis 60 Gew.%, bezogen auf die gesamte Masse, vorliegen; in Druckfarben liegen meist 10–30% Pigment vor.

Ausser den drei essentiellen Komponenten (Bindemittel, Pigment, Photoinitiator) kann die photopolymerisierbare Masse weitere Bestandteile enthalten, die vor allem vom beabsichtigten Verwendungsgebiet abhängen. Während für die meisten Zwecke lösungsmittelfreie Massen bevorzugt sind, kann zur Erzielung der für den Auftrag benötigten Viskosität der Zusatz eines Lösungsmittels notwendig sein. Hierzu sind die üblichen Lacklösungsmittel geeignet, die häufig Gemische verschiedener Lösungsmittel darstellen. Für einen gleichmässigen Auftrag können auch Verlaufshilfsmittel, Thioxotropiemittel oder Netzmittel zugesetzt werden. Bei Druckfarben werden häufig Wachse oder sonstige Gleitmittel zuge-

setzt. Die photopolymerisierbare Masse kann auch als wässrige Dispersion oder Lösung vorliegen.

Obwohl die erfindungsgemässen Massen eine ausgezeichnete Dunkellagerbeständigkeit haben, kann es für bestimmte Zwecke, z.B. für die Verwendung in tropischen Ländern, sinnvoll sein, Polymerisationsinhibitoren zuzusetzen. Hierzu werden z.B. Hydrochinon und dessen Derivate, β-Naphthole, sterisch gehinderte Phenole, Kupferverbindungen, Verbindungen des dreiwertigen Phosphors, Phenothiazin, quartäre Ammoniumverbindungen oder Hydroxylaminderivate verwendet.

Umgekehrt können zur Beschleunigung der UV-Härtung oder Erreichung bestimmter physikalischer Eigenschaften Kettenübertragungsmittel wie tertiäre Amine oder Thiolverbindungen zugesetzt werden. Auch der Zusatz radikalischer Initiatoren wie von Peroxiden oder anderen organischen Perverbindungen sowie von Benzpinakol oder anderen thermisch spaltbaren organischen Verbindungen kann in bestimmten Fällen die Photopolymerisation beschleunigen.

Die erfindungsgemässen Massen können auch einen Photosensibilisator enthalten, der die spektrale Empfindlichkeit in bestimmte Bereiche verschiebt. Dies kann z.B. ein organischer Farbstoff, Perylen oder ein Derivat des Anthracens oder Thioxanthons sein. Insbesondere Thioxanthonderivate, wie z.B. Alkylthioxanthone oder Thioxanthoncarbonsäureester, bewirken als Sensibilisatoren eine starke Beschleunigung der Photopolymerisation.

Bevorzugt verwendet man als Photoinitiator nur eine Verbindung der Formel I. Für spezielle Fälle kann aber die Verwendung eines Gemisches von zwei solchen Verbindungen oder eines Gemisches mit einem anderen bekannten Photoinitiator von Vorteil sein. Die benötigte Menge Photoinitiator in der photohärtbaren gefärbten Masse beträgt 0,1–20 Gew.%, vorzugsweise 1–6 Gew.%.

Die erfindungsgemässen Massen können für verschiedene Zwecke verwendet werden. Die wichtigste und bevorzugte Verwendung ist diejenige für Druckfarben. Hierbei kann es sich z.B. um solche für Offsetdruck, Hochdruck, Tiefdruck, Siebdruck oder Flexodruck handeln. Besonders geeignet sind die erfindungsgemässen Druckfarben für den Offsetdruck, Siebdruck und Tiefdruck.

Ein zweites wichtiges Verwendungsgebiet ist die Verwendung für Anstrichstoffe. Pigmentierte Anstriche finden vor allem als Grundierung für den Korrosionsschutz von Metallen Verwendung, jedoch auch als farbige Decklacke für dekorative Zwecke auf allen möglichen Substraten wie z.B. Metall, Holz, Pappe, Kunststoff oder Textilien. Von besonderem Interesse ist die Anwendung der erfindungsgemässen Massen für Weisslacke und für schwarzpigmentierte Metallprimer. Wässrige Systeme können auch als elektrophoretisch abscheidbare Lacke verwendet werden.

Weitere Verwendungsgebiete sind die Strahlenhärtung von Photoresists, die Photovernetzung silberfreier Filme oder sonstige Gebiete der Photoreproduktion.

In all diesen Verwendungen wird die photohärtbare Masse in dünner Schicht auf ein Substrat aufgetragen. Falls ein Lösungsmittel enthalten war, wird dieses anschliessend weitgehend entfernt, beispielsweise durch Erwärmen in einem Trockenofen, durch Darüberleiten von warmer Luft oder durch Infrarot- oder Mikrowellen-Bestrahlung. Die getrocknete Schicht wird dann mit kurzwelligem Licht bestrahlt, vorzugsweise mit UV-Licht vom Wellenlängenbereich 250–400 nm. Als Lichtquellen sind hierzu z.B. Quecksilbermitteldruck-, -hochdruck und niederdruckstrahler sowie superaktinische Leuchtstoffröhren geeignet. Vorzugsweise wird die Strahlungshärtung in kontinuierlichem Verfahren durchgeführt, wobei das zu härtende Material unter der Strahlenquelle vorbeitransportiert wird. Die Transportgeschwindigkeit ist massgeblich für die Produktionsgeschwindigkeit des Artikels; sie hängt von der benötigten Bestrahlungszeit ab. Deshalb ist die Beschleunigung der Strahlenhärtung durch Photoinitiatoren ein wichtiger Faktor für die Produktion solcher Artikel und es ist einer der Vorteile der Photoinitiatoren der Formel I, dass sie bereits in niedriger Konzentration auch bei Massen mit hohem Pigmentgehalt eine rasche Härtung gewährleisten.

Bei Verwendung eines Hybrid-Systems als Bindemittel kann die Härtung des Filmes in zwei Stufen durchgeführt werden. Beispielsweise wird durch Strahlenpolymerisation der photopolymerisierbaren Anteile ein Prepolymer geschaffen, das anschliessend durch thermische Kondensation der kondensierbaren Anteile ausgehärtet wird. Eine solche zweistufige Verfahrensweise kann z.B. für Beschichtungen oder Verklebungen von Interesse sein sowie bei der Härtung von relativ dicken Schichten.

Ein anderes zweistufiges Verfahren ist die Kombination von Elektronenbestrahlung und UV-Bestrahlung, die ebenfalls für dickere Schichten von Interesse ist. Während die Elektronenstrahlen eine Härtung in der Tiefe des Filmes bewirken, wird die Oberfläche durch die UV-Bestrahlung gehärtet.

Gegenstand der Erfindung ist daher auch ein Verfahren zur photochemischen Härtung von gefärbten Massen, die ein olefinisch ungesättigtes, photopolymerisierbares Bindemittel, ein Pigment und einen Photoinitiator enthalten, durch Bestrahlung mit kurzwelligem Licht, dadurch gekennzeichnet, dass man als Photoinitiator eine Verbindung der Formel I verwendet.

Die folgenden Beispiele erläutern die Herstellung und Verwendung der Verbindungen der Formel I näher. Teile sind darin Gewichtsteile, Prozente sind Gewichts-Prozente und die Temperaturen sind in °C angegeben.

Beispiel 1

Herstellung der Aminoketone über die α-Halogenketone

1a) 4-Methoxy-isobutyrophenon

160 g (1,2 Mol) fein gepulvertes Aluminiumchlorid werden in 500 ml Tetrachlorethylen vorgelegt und auf 0–5° abgekühlt. Zu der weissen Suspension tropft man ein Gemisch von 108 g (1,0 Mol) Anisol und 117 g (1,1 Mol) Isobuttersäurechlorid. Bei unveränderter Temperatur lässt man anschliessend 3 Stunden unter heftigem Rühren ausreagieren und giesst die dickflüssige, gelbliche Suspension dann auf Eis/Wasser. Die organische Phase wird abgetrennt, über Natriumsulfat getrocknet und am Rotationsverdampfer eingeengt. Der Rückstand wird durch Vakuumdestillation gereinigt.

Man erhält 171 g (96% d.Th.) farblose Flüssigkeit mit einem Siedepunkt von 84° bei 3,4 mbar.

Das NMR-Spektrum ist mit der angegebenen Struktur verträglich.

NMR ($CDCl_3$), δ(ppm):

1,17 (d, 6H, J = 7 Hz); 3,43 (m, 1H); 3,73 (s, 3H); 6,77 und 7,77 (AA'BB'-System, $J_{AB}$ = 9 Hz, $J_{AB'} \leqslant 1$ Hz, 4H).

1b) 4-Methoxy-α-bromisobutyrophenon

178 g (1 Mol) 4-Methoxyisobutyrophenon werden in 300 ml Chlorbenzol vorgelegt und bei Raumtemperatur unter Rühren tropfenweise mit 160 g (1 Mol) Brom versetzt. Nach beendetem Zutropfen lässt man 3 Stunden ausreagieren und treibt anschliessend Reste des entstandenen HBr-Gases mit einem Stickstoffstrom aus der Lösung aus. Das Lösungsmittel wird dann am Rotationsverdampfer bei einer Badtemperatur von 40° entfernt.

Als Rückstand bleiben 257 g (100% d.Th.) des α-Bromketons als gelbbraunes Öl, das ohne weitere Reinigung für die nächste Umsetzung verwendet wird.

Das NMR-Spektrum des Rohprodukts ist mit der angegebenen Struktur verträglich.

NMR ($CDCl_3$), δ (ppm):

1,99 (s, 6H); 3,72 (s, 3H); 6,71 und 8,01 (AA'BB'-System, $J_{AB}$ = 9 Hz, $J_{AB'} \leqslant 1$ Hz, 4H).

1c) 2-Methoxy-2-(4-methoxyphenyl)-3,3-dimethyloxiran

Eine Lösung von 70 g (1,3 Mol) Natriummethoxid in 170 g Methanol wird bei Raumtemperatur vorgelegt und unter heftigem Rühren mit 257 g (1 Mol) des rohen Bromketons aus 1b) tropfenweise versetzt. Die Temperatur wird durch leichtes Kühlen auf 20–25° gehalten. Nach beendetem Zutropfen wird 2 Stunden nachgerührt, die entstandene weisse Suspension mit 200 ml Toluol verdünnt und das ausgefallene Natriumbromid abfiltriert. Das Filtrat wird am Rotationsverdampfer eingeengt, wobei weiteres Natriumbromid ausfällt. Nach neuerlicher Filtration wird das Rohprodukt durch Vakuumdestillation gereinigt.

Man erhält 180 g (86% d.Th.) des Epoxyethers als farblose Flüssigkeit, die bei 64°/2,5 mbar siedet. Smp. 41–43°. Das NMR-Spektrum ist mit der angegebenen Struktur verträglich.

NMR ($CDCl_3$), δ (ppm):

0,97 (s, 3H); 1,49 (s, 3H); 3,10 (s, 3H); 3,70 (s, 3H); 6,73 und 7,19 (AA'BB'-System, $J_{AB}$ = 8,5 Hz, $J_{AB'} \leqslant 1$ Hz, 4H).

1d) 1-(4-Methoxyphenyl-2-methyl-2-morpholino-propanon-1 (Verbindung Nr. 1)

208 g (1 Mol) des Epoxyethers aus 1c) werden in 348 g (4 Mol) Morpholin während 12 Stunden zum Rückfluss erhitzt, wobei das entstehende Methanol kontinuierlich abdestilliert. Die erkaltete Lösung wird am Rotationsverdampfer eingeengt und der Rückstand aus Hexan umkristallisiert.

Es werden 237 g des reinen Produktes als farblose Kristalle vom Smp. 75–76° erhalten (Verbindung Nr. 1).

Das NMR-Spektrum ist mit der angegebenen Struktur verträglich.

NMR ($CDCl_3$) δ (ppm):

1,28 (s, 6H); 2,52 (m, 4H); 3,61 (m, 4H); 3,77 (s, 3H); 6,73 und 8,41 (AA'BB'-System, $J_{AB}$ = 9 Hz, $J_{AB'} \leqslant 1$ Hz, 4H).

| Elementaranalyse: | C | H | N | O |
|---|---|---|---|---|
| berechnet | 68,42 | 8,04 | 5,32 | 18,23% |
| gef. | 68,19 | 8,06 | 5,33 | 18,25% |

In analoger Weise wurden die in Tabelle 1 aufgeführten Aminoketone hergestellt.

Tabelle 1

| Verbindung Nr. | Formel | Physikal. Daten | Elementaranalyse (%) C | H | N |
|---|---|---|---|---|---|
| 1 | 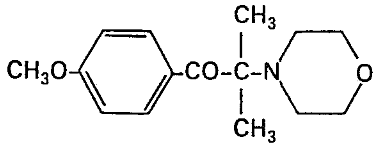 | Smp. 75–76° | ber. 68,42<br>gef. 68,19 | 8,04<br>8,06 | 5,32<br>5,33 |
| 2 | 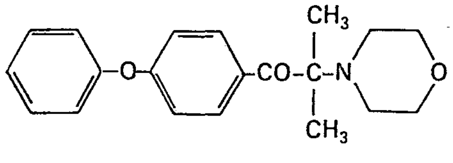 | Smp. 99–101° | ber. 73,82<br>gef. 73,51 | 7,13<br>7,01 | 4,31<br>4,42 |

Tabelle 1 (Fortsetzung)

| Verbindung Nr. | Formel | Physikal. Daten | Elementaranalyse (%) C | H | N |
|---|---|---|---|---|---|
| 3 | 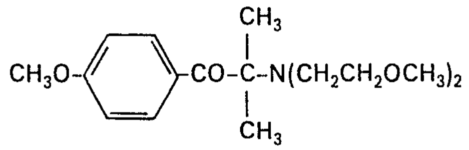 | flüssig | ber. 65,99<br>gef. 65,93 | 8,80<br>8,76 | 4,53<br>4,48 |
| 4 | 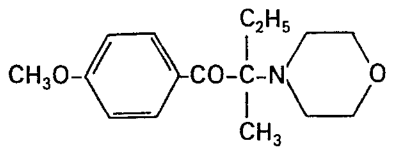 | viskos | ber. 69,29<br>gef. 69,08 | 8,36<br>8,38 | 5,05<br>5,09 |
| 5 | 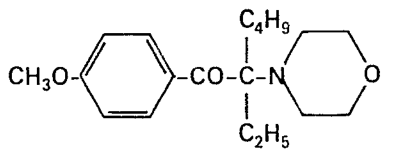 | Smp. 74–76° | ber. 71,44<br>gef. 71,39 | 9,15<br>9,03 | 4,38<br>4,26 |
| 6 | 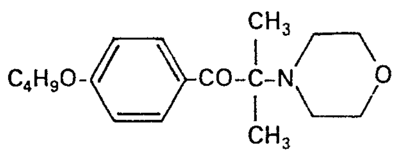 | Smp. 91–93° | ber. 70,79<br>gef. 70,77 | 8,91<br>8,86 | 4,59<br>4,65 |

1e) Chlorierung mit Kupfer-II-chlorid

1-(3,4-Dimethoxyphenyl)-2-chlor-2-methyl-propanon-1

79,1 g (0,38 Mol) 1-(3,4-Dimethoxyphenyl-2-methylpropanon-1, hergestellt analog Beispiel 1a), werden in 400 ml Isopropanol-Wasser-Gemisch 4:1 gelöst. Dann werden 64,8 g (0,38 Mol) Kupfer-II-chlorid (Dihydrat) zugegeben. Die Suspension wird auf Rückfluss (ca. 80°) geheizt. Die grüne Lösung wird dunkler. Nach 8 Stunden werden weitere 64,8 g (0,38 Mol) Kupfer-II-chlorid zugegeben. Die dunkelgrüne Suspension wird weitere 21 Stunden am Rückfluss gehalten. Dann wird gekühlt und filtriert. Das Filtrat wird mit 300 ml Toluol verdünnt, und die Toluollösung wird dreimal mit 200 ml Wasser extrahiert. Die Toluollösung wird mit Natriumsulfat getrocknet und eingeengt. Das erhaltene Rohprodukt wird durch Gaschromatographie und NMR-Spektrum überprüft und dann analog Beispiel 1c) zum entsprechenden Oxiran weiterverarbeitet.

Beispiel 2

Herstellung des freien Phenols durch Etherspaltung

1-(4-Hydroxyphenyl)-2-methyl-2-morpholino-propanon-1

131,7 g (0,5 Mol) 1-(4-Methoxyphenyl)-2-methyl-2-morpholino-propanon-1 werden in 300 ml Bromwasserstoffsäure (ca. 47%) gelöst und während 24 Stunden bei 120°C gerührt. Dann wird die Lösung abgekühlt und mit konzentrierter Natronlauge bis pH 8,5 neutralisiert. Die entstehende Suspension wird mehrmals mit Diethylether extrahiert. Der Ether wird getrocknet und eingeengt. Die zurückbleibenden Kristalle werden aus einem Methanol-Wasser-Gemisch (5:1) umkristallisiert.

Es resultieren 89 g farblose Kristalle vom Smp. 189–192°C.

Das NMR-Spektrum ist mit der angegebenen Struktur verträglich.

NMR ($CDCl_3$/$CH_3OD$), δ (ppm):

1,30 (s, 6H); 2,55 (m, 4H); 3,64 (m, 4H); 4,32 (s, breit, 1H); 6,69 und 8,31 (AA'BB'-System, $J_{AB}$ = 9 Hz, $J_{AB'} \leqslant$ 1 Hz, 4H).

| Elementaranalyse: | C | H | N |
|---|---|---|---|
| berechnet | 67,45 | 7,65 | 5,62% |
| gefunden | 67,47 | 7,67 | 5,61% |

Beispiel 3

Veretherung des freien Phenols

1-[4-(Allyloxy)phenyl]-2-methyl-2-morpholino-propanon-1

Zu einer Suspension von 12,5 g (0,05 Mol) 1-(4-Hydroxyphenyl)-2-methyl-2-morpholino-propanon-1 und 7,6 g pulverisiertem Kaliumcarbonat in 30 ml Aceton werden bei Raumtemperatur 6,7 g (0,055 Mol) Allylbromid zugetropft. Dann wird auf 60°C geheizt und über Nacht bei 60°C gerührt. Dann wird die Suspension gekühlt, mit 100 ml Toluol verdünnt und filtriert. Die Lösung wird am Vacuumrotationsverdampfer eingeengt und erneut mit 100 ml Toluol verdünnt. Die Toluollösung wird mit Wasser extrahiert, mit Kaliumcarbonat getrocknet und am Vacuumrotationsverdampfer eingeengt.

Es werden 14 g eines gelben Öls erhalten (Verbindung Nr. 7).

Das NMR-Spektrum ist mit der angegebenen Struktur verträglich.

NMR ($CCl_4$), δ (ppm):

1,21 (s, 6H); 2,42 (m, 4H); 3,50 (m, 4H); 4,35-4,58 (m, 2H); 4,98-5,45 (m, 2H); 5,58-6,23 (m, 1H); 6,69 und 8,32 (AA'BB'-System, $J_{AB}=9$ Hz, $J_{AB'} \leqslant 1$ Hz, 4H).

| Elementaranalyse: | C | H | N |
|---|---|---|---|
| berechnet | 70,56 | 8,01 | 4,84% |
| gefunden | 70,56 | 8,09 | 4,83% |

Beispiel 4

Verwendung in einer Druckfarbe

Eine blaue Druckfarbe wird nach folgender Rezeptur hergestellt: 62 Teile Setalin® AP 565 (Urethanacrylatharz der Fa. Synthese, Holland),

15 Teile 4,4'-Di-(β-acrylolyloxyethoxy)-diphenylpropan-2,2 (Ebecryl® 150, UCB, Belgien),

23 Teile Irgalithblau® GLSM (Ciba-Geigy AG, Basel).

Das Gemisch wird auf einem 3-Walzen-Stuhl homogenisiert und bis auf eine Korngrösse von <5 µm gemahlen.

Von dieser Druckfarbe werden jeweils 5 g mit der gewünschten Menge an Photoinitiator auf einer Tellerreibmaschine unter einem Druck von 1,7 kPa (180 kg/m²) unter Wasserkühlung homogen vermischt.

Von dieser Druckfarbe werden mit einem Probedruckgerät (Fa. Prüfbau, BR Deutschland) Offsetdrucke auf 4×20-cm-Streifen aus Kunstdruckpapier gemacht. Die Druckbedingungen sind

Auflage Druckfarbe 1,5 g/m²

Anpressdruck 2452 kPa

Druckgeschwindigkeit 1 m/s

Hierbei wird eine Druckwalze mit Metalloberfläche (Aluminium) verwendet.

Die bedruckten Proben werden in einem UV-Bestrahlungsgerät (QC-Processor der Fa. RPC, USA) mit zwei Lampen von je 80 W/cm und einem Abstand zur Lampe von 11 cm bestrahlt. Die Bestrahlungszeit wird durch Variation der Transportgeschwindigkeit der Proben variiert.

Die Oberflächentrocknung der Druckfarben wird unmittelbar nach der Bestrahlung durch den sogenannten Transfer-Test geprüft. Dabei wird ein weisses Papier unter einem Druck von 2452 kPa an die bedruckte Probe angepresst. Wenn das Papier farblos bleibt, ist der Test bestanden. Wenn sichtbare Mengen Farbe auf den Teststreifen übertragen werden, so ist dies ein Zeichen, dass die Oberfläche der Probe noch nicht genügend gehärtet ist.

In der Tabelle 2 ist die maximale Transportgeschwindigkeit angegeben, bei der der Transfer-Test noch bestanden wurde.

Zur Prüfung der Durchhärtung der Druckfarbe werden ebenfalls Offset-Drucke hergestellt, wie vorhin beschrieben, jedoch werden Druckwalzen mit Gummi-Oberfläche verwendet, und es wird die Metallseite von aluminiumbeschichteten Papierstreifen bedruckt.

Die Bestrahlung geschieht wie oben beschrieben. Unmittelbar nach der Bestrahlung wird die Durchhärtung in einem REL-Durchhärtungsprüfgerät getestet. Dabei wird auf die bedruckte Probe ein mit Stoff überspannter Aluminium-Zylinder aufgesetzt und unter einem Druck von 21,6 kPa innerhalb 10 s einmal um die eigene Achse gedreht. Wenn dabei auf der Probe sichtbare Beschädigungen entstehen, so ist die Druckfarbe ungenügend durchgehärtet. In der Tabelle 2 wird die maximale Transportgeschwindigkeit angegeben, bei der der REL-Test noch bestanden wurde.

Tabelle 2

| Photoinitiator | | Maximale Transportgeschwindigkeit (m/min) | |
|---|---|---|---|
| Verbindung Nr. | Menge (Gew.%) | Transfer-Test (Oberflächenhärtung) | REL-Test (Durchhärtung) |
| 1 | 3 | >170 | 120 |
| | 6 | >170 | >170 |
| 3 | 3 | >170 | 80 |
| | 6 | >170 | 160 |
| 4 | 3 | >170 | 90 |
| | 6 | >170 | >170 |
| 7 | 3 | >170 | 90 |
| | 6 | >170 | 150 |

Beispiel 5

Mitverwendung von Thioxanthonen als Sensibilisatoren.

Es wird ein Weisslack nach folgender Rezeptur hergestellt:

17,6 g Ebecryl® 593 (Polyesteracrylatharz der Fa. UCB, Belgien),

11,8 g N-Vinylpyrrolidon

19,6 g Titandioxid RTC-2 (Titandioxid der Fa. Tioxide, England),

19,6 g Sachtolith® HDA (Lithopone der Sachtleben Chemie, BRD)

11,8 g Trimethylolpropan-trisacrylat,

19,6 g Setalux® UV 2276 (acryliertes Epoxidharz auf der Basis von Bisphenol A, Kunstharzfabrik Synthese, Holland).

Die obigen Komponenten werden zusammen mit 125 g Glasperlen (Durchmesser 4 cm) in einer 250-ml-Glasflasche während mindestens 24 Stunden auf eine Korngrösse ≤5 µm gemahlen.

Die so erhaltene Stammpaste wird in Portionen geteilt und jede Portion mit den in der Tabelle 3 angegebenen Photoinitiatoren und Photosensibilisatoren (Co-Initiatoren) durch Einrühren bei 60°C gemischt und die Mischungen nochmals 16 Stunden mit Glasperlen gemahlen.

Die so hergestellten Weisslacke werden in einer Dicke von 30 µm mit einem Rakel auf Glasplatten aufgetragen. Die Proben werden in einem PPG-Bestrahlungsgerät mit einer bzw. zwei Lampen von je 80 W/cm in einem Durchgang belichtet. Dabei wird die Geschwindigkeit des Durchganges der Proben durch das Bestrahlungsgerät laufend gesteigert, bis keine ausreichende Härtung mehr eintritt. Die maximale Geschwindigkeit, bei der noch ein wischfester Lackfilm entsteht, ist in Tabelle 3 als «Härtungsgeschwindigkeit» angegeben.

Hierbei werden folgende Verbindungen verwendet:

Photoinitiator

PI 1 = 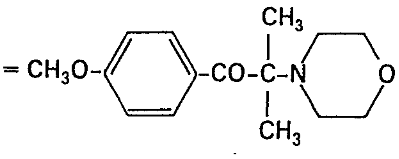

Photosensibilisatoren

PS 1 = 2-Isopropylthioxanthon

PS 2 = 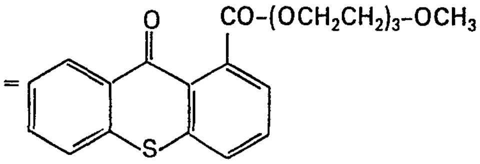

PS 3 = 2-Methyl-6-ethoxycarbonyl-thioxanthon

PS 4 = 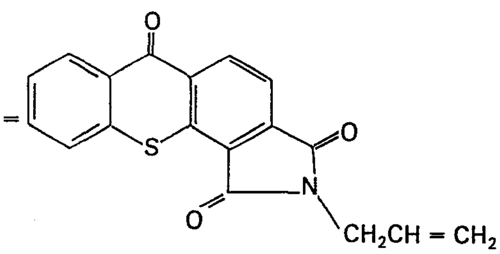

Tabelle 3

| Photoinitator | Coinitiator (Sensibilisator) | Maximale Härtungsgeschwindigkeit bei 80 W/cm | 160 W/cm |
|---|---|---|---|
| 2% PI 1 | - | 10 m/min | 10 m/min |
| 2% PI 1 | 0,25% PS 1 | 30 m/min | 70 m/min |
| 2% PI 1 | 0,25% PS 2 | 20 m/min | 40 m/min |
| 2% PI 1 | 0,25% PS 3 | 70 m/min | 150 m/min |
| 2% PI 1 | 0,25% PS 4 | 90 m/min | >170 m/min |

Man ersieht daraus, dass bereits kleine Mengen an Sensibilisator die Härtungsgeschwindigkeit wesentlich steigern.

**Patentansprüche**

1. Verbindung der Formel I

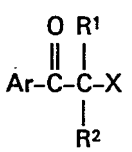 (I)

worin Ar ein in 4-Stellung durch eine Gruppe $-OR^9$ substituierter Phenylrest ist, $R^9$ $C_1$–$C_8$-Alkyl, $C_3$–$C_6$-Alkenyl, Cyclohexyl, Benzyl, Phenyl, Tolyl oder eine der Gruppen $-CH_2CH_2OH$, $-CH_2CH_2-OOC-CH=CH_2$, $-CH_2-COO(C_1-C_4\text{-Alkyl})$, $-CH_2CH_2-COO(C_1-C_4\text{-Alkyl})$,

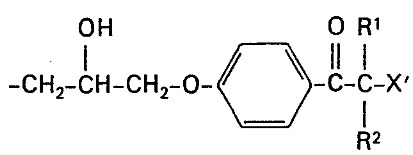

oder 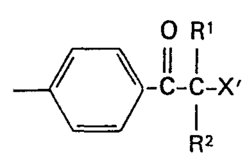

bedeutet, $R^1$ und $R^2$ $C_1$–$C_4$-Alkyl oder $R^1$ und $R^2$ zusammen $C_4$–$C_5$-Alkylen bedeuten und X und X′

ein Morpholinorest oder ein Rest der Formel $-N(CH_2CH_2-OCH_3)_2$ sind.

2. Verbindung gemäss Anspruch 1 der Formel

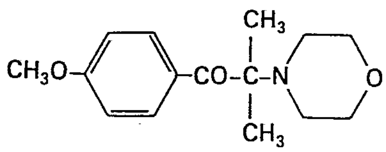

3. Verbindung gemäss Anspruch 1 der Formel

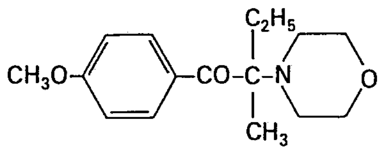

4. Verbindung gemäss Anspruch 1 der Formel

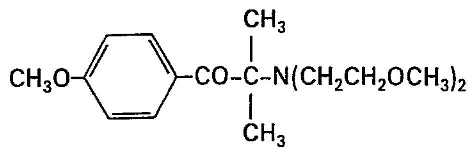

5. Photohärtbare gefärbte Masse, enthaltend a) ein olefinisch ungesättigtes, photopolymerisierbares Bindemittel, b) ein Pigment und c) als Photoinitiator eine Verbindung der Formel I nach Anspruch 1.

6. Masse gemäss Anspruch 5, enthaltend zusätzlich als Sensibilisator ein Thioxanthon-Derivat.

7. Verwendung der photohärtbaren gefärbten Masse nach Anspruch 5 als Druckfarbe.

8. Verfahren zur photochemischen Härtung von gefärbten Massen, die ein olefinisch ungesättigtes, photopolymerisierbares Bindemittel, ein Pigment und ein Photoinitiator enthalten, durch Bestrahlung mit kurzwelligem Licht, dadurch gekennzeichnet, dass man als Photoinitiator eine Verbindung der Formel I gemäss Anspruch 1 verwendet.

**Claims**

1. A compound of formula I

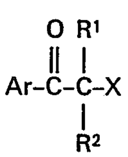 (I)

wherein Ar is phenyl which is substituted in 4-position by an $-OR^9$ group, in which $R^9$ is $C_1$–$C_8$ alkyl, $C_3$–$C_6$ alkenyl, cyclohexyl, benzyl, phenyl, tolyl or a group selected from $-CH_2CH_2OH$, $-CH_2CH_2-OOC-CH=CH_2$, $-CH_2-COO(C_1-C_4\ alkyl)$, $-CH_2CH_2-COO(C_1-C_4\ alkyl)$,

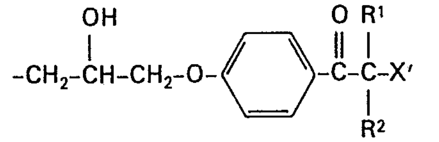

or

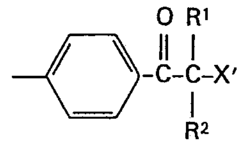

$R^1$ and $R^2$ are each $C_1$–$C_4$ alkyl or together are $C_4$–$C_5$ alkylene, and X and X′ are a morpholino radical or a radical of formula $-N(CH_2CH_2-OCH_3)_2$.

2. A compound according to claim 1, of formula

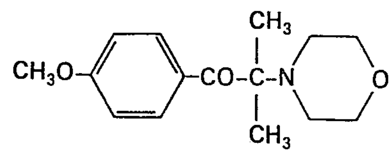

3. A compound according to claim 1, of formula

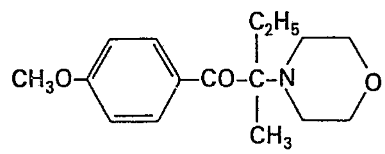

4. A compound according to claim 1, of formula

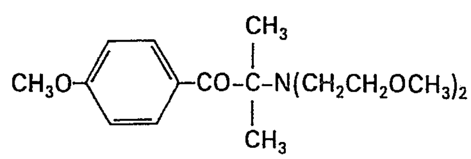

5. A photocurable coloured composition comprising a) an olefinically unsaturated photopolymerisable binder, b) a pigment, and c) a compound of formula I according to claim 1 as photoinitiator.

6. A composition according to claim 5, which additionally comprises a thioxanthone derivative.

7. Use of a photocurable coloured composition according to claim 5 as printing ink.

8. A process for the photochemical curing of coloured compositions containing an olefinically unsaturated, photopolymerisable binder, a pigment and a photoinitiator, by irradiation with shortwave light, which process comprises using a compound of formula I according to claim 1 as photoinitiator.

**Revendications**

1. Composés répondant à la formule I:

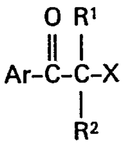

(I)

dans laquelle Ar représente un radical phényle qui porte, en position 4, un radical $-OR^9$ dont le symbole $R^9$ désigne un alkyle en $C_1$–$C_8$, un alcényle en $C_3$–$C_6$, un cyclohexyle, un benzyle, un phényle, un tolyle ou un radical répondant à l'une des formules $-CH_2CH_2OH$, $-CH_2CH_2-OOC-CH=CH_2$, $-CH_2-COO(C_1-C_4\text{-alkyl})$, $-CH_2CH_2-COO(C_1-C_4\text{-alkyl})$,

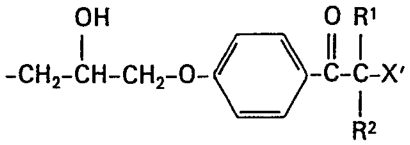

et

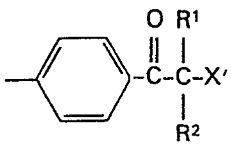

$R^1$ et $R^2$ représentent chacun un alkyle en $C_1$–$C_4$ ou forment ensemble un alkylène en $C_4$ ou $C_5$, et X et X′ représentent chacun un radical morpholino ou un radical $-N(CH_2CH_2-OCH_3)_2$.

2. Composé selon la revendication 1 qui répond à la formule suivante:

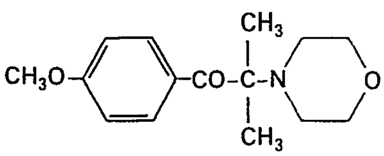

3. Composé selon la revendication 1 qui répond à la formule suivante:

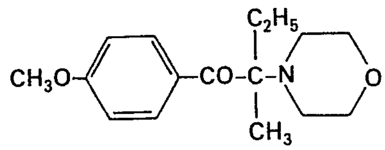

4. Composé selon la revendication 1 qui répond à la formule suivante:

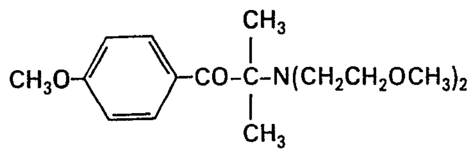

5. Matière colorée photodurcissable qui contient: a) un liant éthylénique photopolymérisable, b) un pigment et c) comme photo-amorceur un composé de formule I selon la revendication 1.

6. Matière selon la revendication 5 qui contient en outre, comme sensibilisateur, un dérivé de la thioxanthone.

7. Application de la matière colorée photodurcissable selon la revendication 5 comme encre d'imprimerie.

8. Procédé pour durcir photochimiquement des matières colorées contenant un liant éthylénique photopolymérisable, un pigment et un photo-amorceur, par exposition à un rayonnement de courte longueur d'onde, procédé caractérisé en ce qu'on utilise, comme photo-amorceur, un composé de formule I selon la revendication 1.